# EUROPÄISCHE PATENTANMELDUNG

(11) **EP 1 502 616 A1**
(43) Veröffentlichungstag der Anmeldung: **02.02.2005**
(21) Anmeldenummer: 03017423.9
(22) Anmeldetag: 01.08.2003
(51) Int. Cl.: A61M 5/32

(54) **Nadelschutz für eine Glasspritze**

(71) Anmelder: Bünder Glas GmbH, 32257 Bünde (DE)
(72) Erfinder: Geiger, Andreas, 32278 Kirchlengern (DE); Stohlmann, Erhard, 32257 Bünde (DE)
(74) Vertreter: Schirmer, Siegfried, Dipl.-Ing.

(57) **Zusammenfassung**

Nadelschutz für eine Glasspritze (2), der einen Verschlussstopfen (3), ein Halteelement (5) mit einer Öffnung zum Aufnehmen eines Abschnitts (6) eines Spritzenkörpers (8) der Glasspritze (2) und ein den Verschlussstopfen (3) und das Halteelement (5) aufnehmendes und die Nadel (4) umschließendes Schutzelement (7) aufweist.

## Beschreibung

Die Erfindung betrifft einen Nadelschutz für eine Glasspritze.

Es ist bekannt, dass Glasspritzen mit Nadeln, die i. A. mit dem Spritzenkörper beispielsweise durch Klebemittel dauerhaft verbunden sind, mit einem aufschiebbaren Nadelschutz aus elastischem Material, wie Gummi, verschlossen werdenkönnen. Dieser Nadelschutz umschließt die Nadel und kann problemlos abgezogen werden. Ein derartiger Nadelschutz wird unter anderem in US 3,865,236 beschrieben.

Probleme treten auf, wenn die Glasspritze unterschiedlichen Temperaturen oder Luftdrücken ausgesetzt wird. In der Regel werden Glasspritzen, nachdem sie mit einer Flüssigkeit, die beispielsweise pharmazeutische Wirkstoffe enthält, gefüllt und mit dem bekannten Nadelschutz verschlossen worden sind, sterilisiert.

Dabei werden sie hohen Temperaturen, zum Beispiel 121°C oder 134°C und einem Vakuum ausgesetzt, so daß sich der elastische Nadelschutz aufbläht. Dies führt häufig zu einer Lockerung des Nadelschutzes und zu einem Abrutschen von der Glasspritze. So kommt es bei der Sterilisation oft zu einem Abspringen des Nadelschutzes von der Glasspritze (engl. pop-off effect).

Es ist die Aufgabe der vorliegenden Erfindung, einen Nadelschutz anzugeben, bei dem die oben beschriebene Lockerung verhindert wird. Weiterhin soll ein Nadelschutz angegeben werden, bei dem eine nachträgliche Manipulation der Spritzenfüllung sofort erkennbar ist.

Diese Aufgabe wird erfindungsgemäß durch einen Nadelschutz für eine Glasspritze gemäß Anspruch 1 gelöst. Vorteilhafte Weiterbildungen der Erfindung sind in den Unteransprüchen aufgezeigt.

Ausführungsbeispiele der Erfindung werden nachfolgend anhand der Zeichnungen näher erläutert. Es zeigen:
- Fig. 1: eine Schnittansicht einer ersten Ausführungsform eines erfindungsgemäßen Nadelschutzes, der auf eine Glasspritze aufgeschoben ist,
- Fig. 2: eine Schnittansicht einer weiteren Ausführungsform eines erfindungsgemäßen Nadelschutzes, der auf eine Glasspritze aufgeschoben ist,
- Fig.: 3 eine Schnittansicht einer weiteren Ausführungsform eines erfindungsgemäßen Nadelschutzes, der auf eine Glasspritze aufgeschoben ist, und
- Fig. 4: eine Schnittansicht einer weiteren Ausführungsform eines erfindungsgemäßen Nadelschutzes, der auf eine Glasspritze aufgeschoben ist.

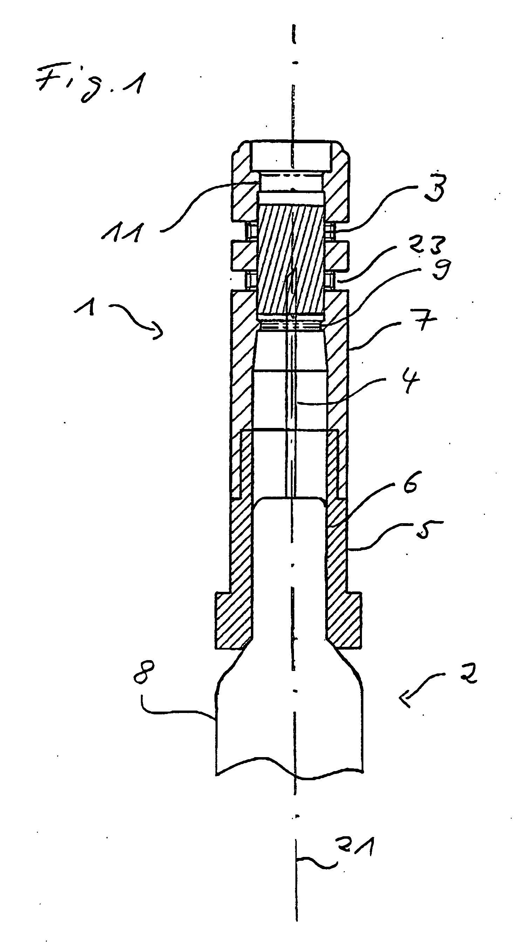

Zunächst wird die Ausführungsform gemäß Fig. 1 beschrieben. Der Nadelschutz 1 weist ein Halteelement 5, ein Schutzelement 7 und einen Verschlussstopfen 3 auf. Das Halteelement 5 besteht aus einem elastischen Material, beispielsweise aus einem thermoplastischen Elastomer. Es ist im wesentlichen in der Form eines Hohlzylinders ausgebildet, der in diesem Ausführungsbeispiel an seinen Außenwänden Stufen aufweist. Im aufgeschobenen Zustand nimmt das zylinderförmige Halteelement 5 einen Abschnitt 6 des Spritzenkörpers 8 der Glasspritze 2 auf. Das Halteelement 5 weist unterschiedliche Außendurchmesser auf, wobei der Außendurchmesser des der Glasspritze zugewandten Endabschnitts am größten und der Außendurchmesser des anderen, gegenüberliegenden Endabschnittes am kleinsten ist. Der Außendurchmesser des zuletzt genannten Endabschnittes des Halteelementes 5 ist so gewählt, daß er von dem Schutzelement 7 aufgenommen werden kann.

Im aufgeschobenen Zustand hält das Halteelement 5 aufgrund seiner Elastizität den Nadelschutz 1 auf der Glasspritze 2 und dichtet den Innenraum des Nadelschutzes 1 ab.

Das Schutzelement 7 besteht aus einem formstabilen Material, wie beispielsweise Polypropylen, das im Vergleich zu dem Halteelement 5 und zu dem Verschlussstopfen 3 kaum elastisch ist. Das Schutzelement 7 ist im wesentlichen als Hohlzylinder ausgebildet, der, wenn der Nadelschutz 1 auf die Glasspritze 2 gesetzt worden ist, die Nadel 4 umschließt. Das zylinderförmige Schutzelement 7 kann entlang seiner Längsachse 21 unterschiedliche Innendurchmesser aufweisen, wobei der Innendurchmesser des dem Halteelement 5 zugewandten Endabschnittes so bemessen ist, daß der entsprechende Endabschnitt des Halteelementes 5 aufgenommen werden kann. Der andere Endabschnitt des Schutzelementes 7 weist einen solchen Innendurchmesser auf, dass der Verschlussstopfen 3 aufnehmbar ist. Das Schutzelement 7 und das Halteelement 5 sind dauerhaft miteinander verbunden, beispielsweise durch Klebemittel, ineinander greifende Rastelemente, Schraubmittel oder ähnliche Verbindungsmittel. Das Schutzelement 7 ist an seinem der Spritze abgewandten Ende offen, könnte aber auch geschlossen sein. Es weist zwei Halterungen 9, 11 auf, die den Verschlussstopfen 3 an einer vorbestimmten Position zwischen den Halterungen 9, 11 halten. Die Halterung 9 wird in diesem Ausführungsbeispiel durch Stege gebildet, die radial in den Innenraum des Schutzelementes 7 hinein ragen, während die Halterung 11 eine Ringwulst ist. Im Bereich des Verschlussstopfens 3 weist das Schutzelement 7 Öffnungen 23 auf, die in diesem Ausführungsbeispiel länglich ausgebildet sind und quer zur Längsachse 21 des im wesentlichen zylinderförmigen Schutzelementes 7 verlaufen. Die Öffnungen 23 haben die Aufgabe, sterilisierende Gase durch Permeation durch den Verschlussstopfen 3 eindringen zu lassen.

Der Verschlussstopfen 3 besteht aus einem elastischen Abdichtungsmaterial, wie beispielsweise Gummi. Der Verschlussstopfen 3 kann gasdurchlässig sein. Der Verschlussstopfen 3 ist zylinderförmig ausgebildet und weist einen Außendurchmesser auf, der im wesentlichen gleich dem Innendurchmesser des dem Spritzenkörper 8 abgewandten Endabschnittes des Schutzelementes 7 ist. Im aufgesetzten Zustand dringt die Nadel 4 der Glasspritze 2 in den Verschlussstopfen 3 ein, so daß die Nadel 4 verschlossen ist.

Da der erfindungsgemäße Nadelschutz 1 zu einem großen Teil aus dem Schutzelement 7 besteht, das im Vergleich zu dem Halteelement 5 und dem Verschlussstopfen 3 kaum elastisch ist, tritt eine unerwünschte Lockerung des Nadelschutzes 1 aufgrund von unterschiedlichen Temperaturen oder Luftdrücken kaum auf.

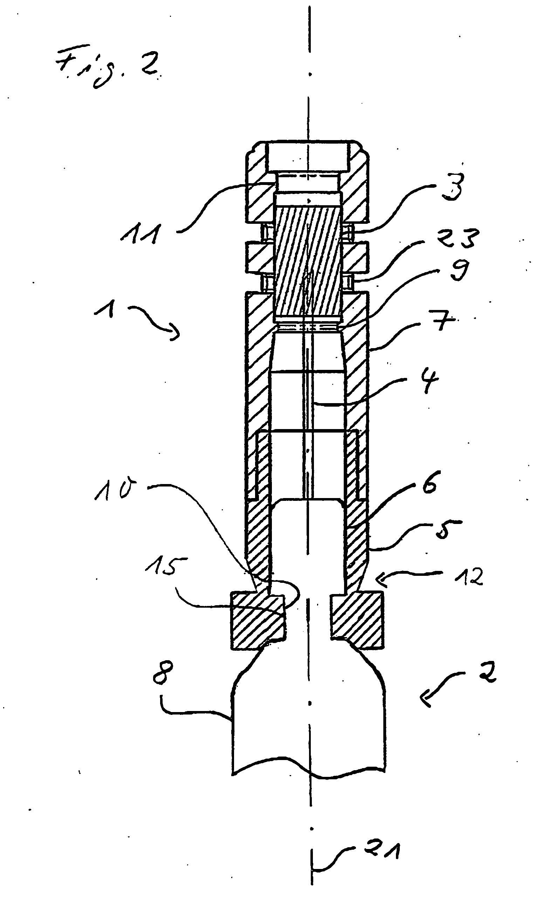

Eine weitere Ausführungsform ist in Fig. 2 dargestellt. Auch hier besteht der erfindungsgemäße Nadelschutz 1 aus einem Halteelement 5, einem Schutzelement 7 und einem Verschlussstopfen 3. Während das Schutzelement 7 und der Verschlussstopfen 3 im Vergleich zur ersten Ausführungsform unverändert sind, weist das Halteelement 5 hier ein Rastelement 15 auf, das in dem in Fig. 2 gezeigten aufgesetzten Zustand mit einem Rastelement 10 des Spritzenkörpers 8 unlösbar verrastet ist. Das Rastelement 15 des Halteelements 5 ist eine Ringwulst 15, und das Rastelement 10 des Spritzenkörpers 8 ist eine ringförmige Erweiterung oder Vertiefung 10. Auf diese Art ist das Halteelement 5 mit dem Spritzenkörper 8 dauerhaft verbunden. In anderen Ausführungsbeispielen könnte das Halteelement 5 auch durch andere Verbindungsmittel, zum Beispiel durch Klebemittel, unlösbar mit dem Spritzenkörper 8 verbunden sein. Das Halteelement 5 weist angrenzend an die dem Spritzenkörper 8 abgewandte Seite der Ringwulst 15 eine Sollbruchstelle 12 auf, die als umlaufende Querschnittsschwächung ausgebildet ist. Die Sollbruchstelle 12 kann auch auf eine andere Art ausgebildet sein, beispielsweise durch Schlitze. Aufgabe der Sollbruchstelle ist die unmittelbare Sichtbarmachung einer Manipulation an der Spritzenfüllung. Beim Abnehmen des Nadelschutzes 1 gemäß dem in Fig. 2 gezeigten Ausführungsbeispiel wird die Ringwulst 15 an der Sollbruchstelle 12 vom dem Halteelement 5 abgetrennt und verbleibt am Spritzenkörper 8. Wird der auf diese Weise beschädigte Nadelschutz 1 wieder auf die Glasspritze 2 aufgeschoben, so ist das vorherige Abnehmen des Nadelschutzes 1 an dem an der Sollbruchstelle 12 durchbrochenen Halteelement 5 erkennbar.

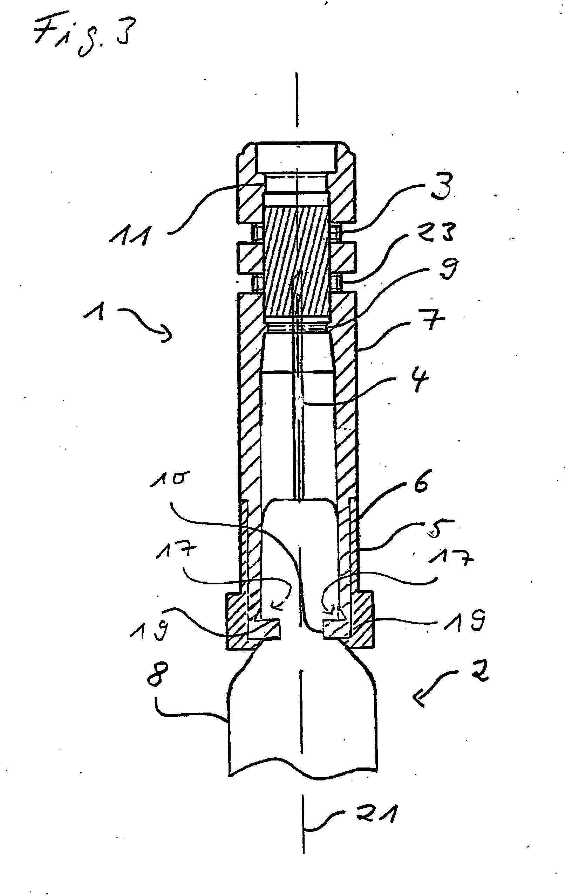

In Fig. 3 ist eine weitere Ausführungsform dargestellt. Auch dort besteht der erfindungsgemäße Nadelschutz 1 aus einem Halteelement 5, einem Schutzelement 7 und einem Verschlussstopfen 3, wobei der Verschlussstopfen 3 im Vergleich zur ersten Ausführungsform unverändert ist. In Fig. 3 weist das im wesentlichen als Hohlzylinder ausgebildete Halteelement 5 in dem dem Spritzenkörper 8 abgewandten Endabschnitt einen Innendurchmesser auf, der im wesentlichen gleich dem Außendurchmesser des dem Spritzenkörper 8 zugewandten Endabschnittes des Schutzelementes 7 ist, so dass der Endabschnitt des Halteelementes 5 den Endabschnitt des Schutzelementes 7 aufnehmen kann. Das Schutzelement 7 ist mit dem Spritzenkörper 8 dauerhaft verbunden. Verbindungsmittel zum Herstellen dieser Verbindung können Klebemittel, Schraubmittel oder andere bekannte Verbindungsmittel sein. In diesem Ausführungsbeispiel ist im aufgesetzten Zustand ein Rastelement 19 des Schutzelementes 7 mit einem Rastelement des Spritzenkörpers 8 verrastet. Das Rastelement des Schutzelementes 7 kann beispielsweise eine Ringwulst 19 sein, und das Rastelement des Spritzenkörpers 8 kann eine ringförmige Erweiterung oder Vertiefung 10 des Spritzenkörpers 8 sein. Das Schutzelement 7 weist angrenzend an die der Nadel 4 zugewandten Seite des Rastelementes 19 eine Sollbruchstelle 17 auf. Die Sollbruchstelle 17 ist in diesem Ausführungsbeispiel als eine umlaufende Querschnittsschwächung ausgebildet. Die Sollbruchstelle könnte auch durch Schlitze ausgebildet sein.

In der Ausführungsform gemäß Fig. 3 liegt das Halteelement 5 an dem Spritzenkörper 8 an und dichtet so den Innenraum des Nadelschutzes 1 ab. Gehalten wird der Nadelschutz 1 auf der Glasspritze 2 hauptsächlich durch das Schutzelement 7, wohingegen das Halteelement 5 weniger zum Halten des Nadelschutzes 1 beiträgt.

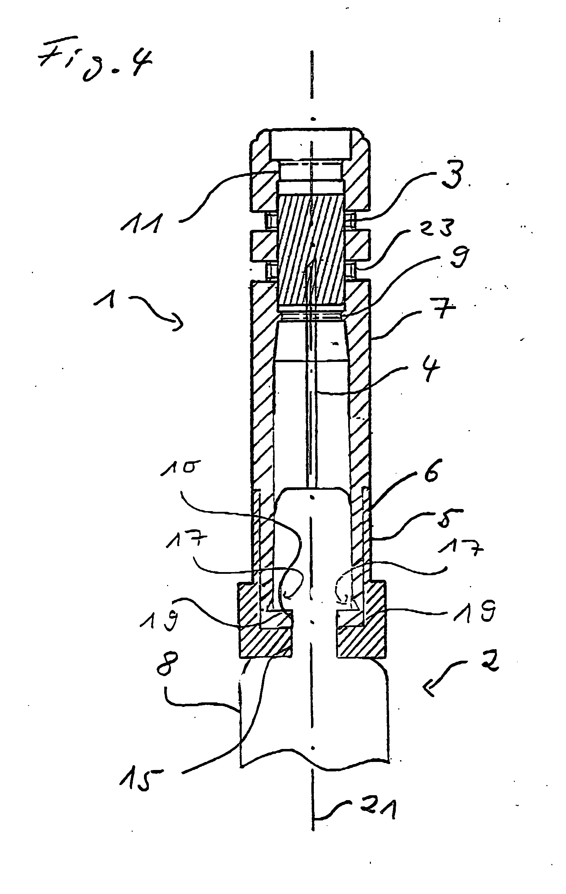

In der Ausführungsform des Nadelschutzes 1 gemäß Fig. 4 sind der Verschlussstopfen 3 und das Schutzelement 7 gegenüber der Ausführungsform gemäß Fig. 3 unverändert. Das Halteelement 5 weist eine ringförmige Wulst 15 auf, die angrenzend an die ringförmige Wulst 19 des Schutzelements 7 mit der ringförmigen Vertiefung 10 des Spritzenkörpers 8 verrastbar ist. Hier dichtet das Halteelement 5 den Innenraum des Nadelschutzes 1 ab. Das Halteelement 5 und das Schutzelement 7 halten beide den Nadelschutz 1 auf den Spritzenkörper 8.

Wird der Nadelschutz 1 gemäß den Ausführungsbeispielen in den Figuren 2 bis 4 von der Glasspritze 2 entfernt, so bricht der Nadelschutz 1 an den jeweiligen Sollbruchstellen 12, 17, wodurch ein Abschnitt des Nadelschutzes 1 an der Glasspritze 2 verbleibt. Dieser Nadelschutz 1 ist also nicht zerstörungsfrei von der Glasspritze 2 lösbar. Wird der Nadelschutz 1 wieder auf die Glasspritze 2 gesetzt, so ist an der gebrochenen Sollbruchstelle 12, 17 sofort erkennbar, dass der Nadelschutz vorher schon einmal von der Glasspritze entfernt worden war. Daher können mit diesem Nadelschutz 1 mögliche Manipulationen an der Spritzenfüllung frühzeitig erkannt werden.

Das Schutzelement 7 kann außenwandige Rippen aufweisen, die beispielsweise parallel zur Längsachse 21 des Schutzelementes 7 angeordnet sein können. Die Rippen verbessern den Griff und erhöhen die Steifigkeit des Schutzelements 7.

### Aufstellung der Bezugszeichen:

- 1: Nadelschutz
- 2: Glasspritze
- 3: Verschlussstopfen
- 4: Nadel
- 5: Halteelement
- 6: Abschnitt des Spritzenkörpers
- 7: Schutzelement
- 8: Spritzenkörper
- 9, 11: Halterungen
- 9: ringförmige Vertiefung
- 12, 17: Sollbruchstelle
- 15, 19: Ringwulst
- 21: Längsachse
- 23: Öffnungen

## Patentansprüche

1. Nadelschutz für eine Glasspritze (2), der einen Verschlussstopfen (3), ein Halteelement (5) mit einer Öffnung zum Aufnehmen eines Abschnitts (6) eines Spritzenkörpers (8) der Glasspritze (2) und ein den Verschlussstopfen (3) und das Halteelement (5) aufnehmendes und die Nadel (4) umschließendes Schutzelement (7) aufweist.

2. Nadelschutz nach einem der vorhergehenden Ansprüche, **dadurch gekennzeichnet, dass** das Halteelement (5) elastisch ist.

3. Nadelschutz nach einem der vorhergehenden Ansprüche, **dadurch gekennzeichnet, dass** das Halteelement (5) im wesentlichen die Form eines Hohlzylinders hat.

4. Nadelschutz nach einem der vorhergehenden Ansprüche, **dadurch gekennzeichnet, dass** das Halteelement (5) außenwandige Stufen aufweist.

5. Nadelschutz nach einem der vorhergehenden Ansprüche, **dadurch gekennzeichnet, dass** ein Endabschnitt des Halteelements (5) dauerhaft mit dem Spritzenkörper (8) verbunden ist.

6. Nadelschutz nach einem der vorhergehenden Ansprüche, **dadurch gekennzeichnet, dass** das Halteelement (5) ein Rastelement aufweist, das mit einem Rastelement des Spritzenkörpers (8) verrastbar ist.

7. Nadelschutz nach Anspruch 6, **dadurch gekennzeichnet, dass** das Rastelement des Halteelements (5) eine nach innen weisende Ringwulst (15) und das Rastelement des Spritzenkörpers (8) eine ringförmige Erweiterung oder Vertiefung (10) ist.

8. Nadelschutz nach einem der Ansprüche 6 oder 7, **dadurch gekennzeichnet, dass** das Rastelement des Halteelements (5) beim Entfernen des Nadelschutzes (1) von der Glasspritze (2) von dem Halteelement (5) abtrennbar ist.

9. Nadelschutz nach einem der vorhergehenden Ansprüche, **dadurch gekennzeichnet, dass** das Halteelement (5) zumindest eine Sollbruchstelle (12) aufweist.

10. Nadelschutz nach Anspruch 9, **dadurch gekennzeichnet, dass** die Sollbruchstelle (12) umlaufend ausgebildet ist.

11. Nadelschutz nach einem der Ansprüche 9 oder 10 soweit rückbezogen auf Anspruch 6, **dadurch gekennzeichnet, dass** die Sollbruchstelle (12) an das Rastelement des Halteelements (5) angrenzt.

12. Nadelschutz nach einem der vorhergehenden Ansprüche, **dadurch gekennzeichnet, dass** das Halteelement (5) im Spritzgießverfahren aus Chemiewerkstoff hergestellt wird.

13. Nadelschutz nach einem der vorhergehenden Ansprüche, **dadurch gekennzeichnet, dass** das Schutzelement (7) nicht elastisch ist.

14. Nadelschutz nach einem der vorhergehenden Ansprüche, **dadurch gekennzeichnet, dass** das Schutzelement (7) im wesentlichen die Form eines Hohlzylinders hat.

15. Nadelschutz nach Anspruch 14, **dadurch gekennzeichnet, dass** das Schutzelement (7) an einem Endabschnitt einen Innendurchmesser aufweist, der den Verschlussstopfen (3) aufnehmen kann.

16. Nadelschutz nach einem der Ansprüche 14 oder 15, **dadurch gekennzeichnet, dass** das Schutzelement (7) an einem Endabschnitt einen Innendurchmesser aufweist. der das Halteelement (5) aufnehmen kann.

17. Nadelschutz nach einem der vorhergehenden Ansprüche, **dadurch gekennzeichnet, dass** ein Endabschnitt des Schutzelements (7) dauerhaft mit dem Spritzenkörper (8) verbunden ist.

18. Nadelschutz nach einem der vorhergehenden Ansprüche, **dadurch gekennzeichnet, dass** das Schutzelement (7) ein Rastelement aufweist, das mit einem Rastelement des Spritzenkörpers (8) verrastbar ist.
